Europäisches Patentamt

European Patent Office

Office européen des brevets

(19)

(11) Veröffentlichungsnummer: **0 135 517**
**B1**

(12) **EUROPÄISCHE PATENTSCHRIFT**

(45) Veröffentlichungstag der Patentschrift:
**22.04.87**

(51) Int. Cl.⁴: **A 61 M 1/28**

(21) Anmeldenummer: **84900605.1**

(22) Anmeldetag: **09.02.84**

(86) Internationale Anmeldenummer:
**PCT/CH 84/00020**

(87) Internationale Veröffentlichungsnummer:
**WO 84/03046 (16.08.84 Gazette 84/20)**

(54) **BEUTELSYSTEM FÜR DIE DURCHFÜHRUNG DER AMBULANTEN PERITONEALDIALYSE.**

(30) Priorität: **10.02.83 CH 768/83**

(43) Veröffentlichungstag der Anmeldung:
**03.04.85 Patentblatt 85/14**

(45) Bekanntmachung des Hinweises auf die Patenterteilung:
**22.04.87 Patentblatt 87/17**

(84) Benannte Vertragsstaaten:
**AT BE CH DE FR GB LI LU NL SE**

(56) Entgegenhaltungen:
**EP - A - 0 049 525**
**EP - A - 0 062 148**
**DE - A - 2 809 303**
**FR - A - 2 486 803**
**GB - A - 2 059 776**
**US - A - 4 306 976**

(73) Patentinhaber: **Laboratorium Dr. G. Bichsel AG,
Bahnhofstrasse 5a, CH-3800 Interlaken (CH)**

(72) Erfinder: **BICHSEL, Guido, Bahnhofstrasse 7,
CH-3800 Interlaken (CH)**

(74) Vertreter: **Gasser, François W.,
Hirschengraben 10 Postfach 1555, CH-3001 Bern (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die vorliegende Erfindung betrifft ein Beutelsystem für die Durchführung der ambulanten Peritonealdialyse.

Bei niereninsuffizienten Patienten wird die Peritonealdialyse als chronische Entgiftungsmethode angewendet. Sie setzt einen offenen Zugang zur Bauchhöhle voraus, was mittels eines Tenckhoff-Katheters erreicht wird. Um der dadurch geschaffenen Gefahr von sekundären Infektionen, Peritonitis genannt, zu begegnen und die Dialyse selber einfach und möglichst risikolos zu gestalten, wurde bis anhin gerne ein Zweibeutelsystem verwendet, wie es beispielsweise in der US-A-4306976 (Bazzato) beschrieben ist. Ein derartiges Beutelsystem weist zwei Beutel auf, die mittels Schläuchen und zwei Y-Verbindungsstücken miteinander und mit dem Tenckhoff-Katheter verbindbar sind. Es ermöglicht das Auswechseln des Dialysats in einem geschlossenen System.

Dieses bekannte Beutelsystem weist allerdings unter anderem den Nachteil auf, das es nicht verhindern kann, dass das frische Dialysat aus dem einen Beutel nach dem Ausfliessen des verschmutzten Dialysats aus der Bauchhöhle des Patienten in den anderen Beutel durch entsprechend verunreinigte Schläuche fliesst, was eine nicht unerhebliche Peritonitis-Restgefahr beinhaltet. Zudem bedingt dieses bekannte Zweibeutelsystem, dass der Tenckhoff-Katheter mit einem Y-Verbindungsstück versehen wird, das der Patient dauernd mit sich herumtragen muss.

Der vorliegenden Erfindung liegt die Aufgabe zugrunde, ein verbessertes Beutelsystem für die Durchführung der ambulanten Peritonealdialyse zu schaffen, das in einem geschlossenen Kreislauf nach dem Ausfliessen des verbrauchten Dialysats aus der Bauchhöhle des Patienten die Sterilisation der verschmutzten Schläuche ermöglicht, derart, dass das Peritonitis-Risiko wesentlich verringert, wenn nicht ganz ausgeschlossen werden kann, und das die Verwendung eines zweiten Y-Verbindungsstückes unnötig macht, so dass der Patient weniger Unannehmlichkeiten hat.

Im folgenden wird das erfindungsgemässe Beutelsystem sowie das Verfahren zu seiner Anwendung anhand der Zeichnung erläutert. In dieser zeigt:

Fig. 1 das Beutelsystem mit seinen einzelnen Komponenten und

Fig. 2 schematisch den Ablauf eines Dialysatwechsels mit dem System nach Fig. 1.

Das Beutelsystem gemäss der Erfindung besteht im wesentlichen aus zwei Beuteln, die über ein Y-Verbindungsstück sowohl miteinander als auch mit einem in die Bauchhöhle des Patienten reichenden Tenckhoff-Katheter verbindbar sind. Im einen Beutel kann die frische Dialyselösung gelagert werden, wogegen das aus der Bauchhöhle zu entnehmende Dialysat in den zweiten Beutel fliessen kann. Eine vor dem Ansetzen der Dialyse in zum ersten Beutel führenden Schlauch gelagerte Desinfektionsflüssigkeit sorgt für absolute Sauberkeit des Systems, bevor die neue Dialyselösung in die Bauchhöhle geleitet wird.

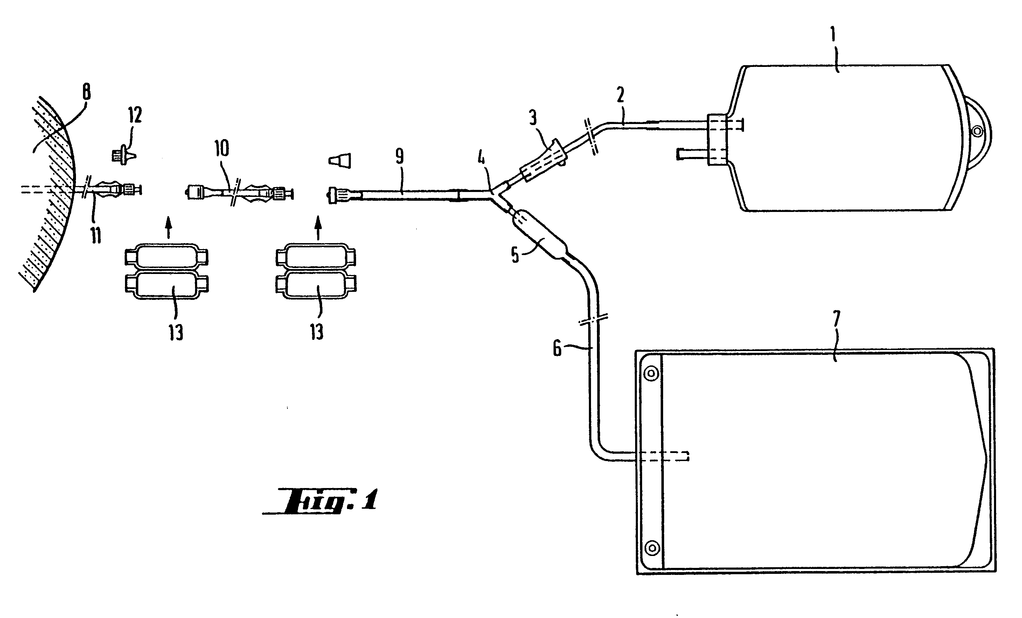

In Fig. 1 ist das Beutelsystem schematisch dargestellt. Der die frische Dialyselösung aufnehmende erste Beutel ist mit der Referenznummer 1 bezeichnet. Er ist über einen ersten Schlauch 2, der, weil er eine genügende Menge von Desinfektionsmittel aufnehmen muss, vorteilhafterweise eine Länge von ca. 1,5 m aufweist und einen bekannten Rollschieber 3 trägt, mit einem Y-Stück 4 verbunden. Letzteres ist seinerseits über eine Tropfkammer 5 und einen zweiten Schlauch 6 mit einem zweiten Beutel 7 verbunden, der das Dialysat aus der Bauchhöhle 8 des Patienten sowie nach der Desinfektion des Systems die Desinfektionslösung aufzunehmen hat. Der dritte Schenkel des Y-Stückes 4 ist über einen dritten Schlauch 9 und ein Katheter-Verlängerungsstück 10 mit dem dem Patienten implantierten Tenckhoff-Katheter 11 verbindbar. Selbstverständlicherweise werden für alle Verbindungen zwischen den Beuteln und Schläuchen und anderen Elementen vorteilhafterweise Elemente des Luer-Lock-Schliess-Systems und Metallkonnektoren verwendet, die in der Medizin seit langem eingeführt sind. Um unerwünschte Infektionskeime am Eindringen in das Peritoneum zu hindern, ist ein Sicherheitsverschluss 12 vorgesehen, der den Tenckhoff-Katheter 11 völlig dicht abschliesst, wenn nicht das erfindungsgemässe Beutelsystem daran angeschlossen ist. Ferner werden während der Dialyse die beiden Verbindungen zwischen Katheter 11 und dem Verlängerungsstück 10 sowie diesem und dem dritten Schlauch 9 vorteilhafterweise durch Protektoren 13, die mit einer Betadine-getränkten Einlage versehen sind, geschützt.

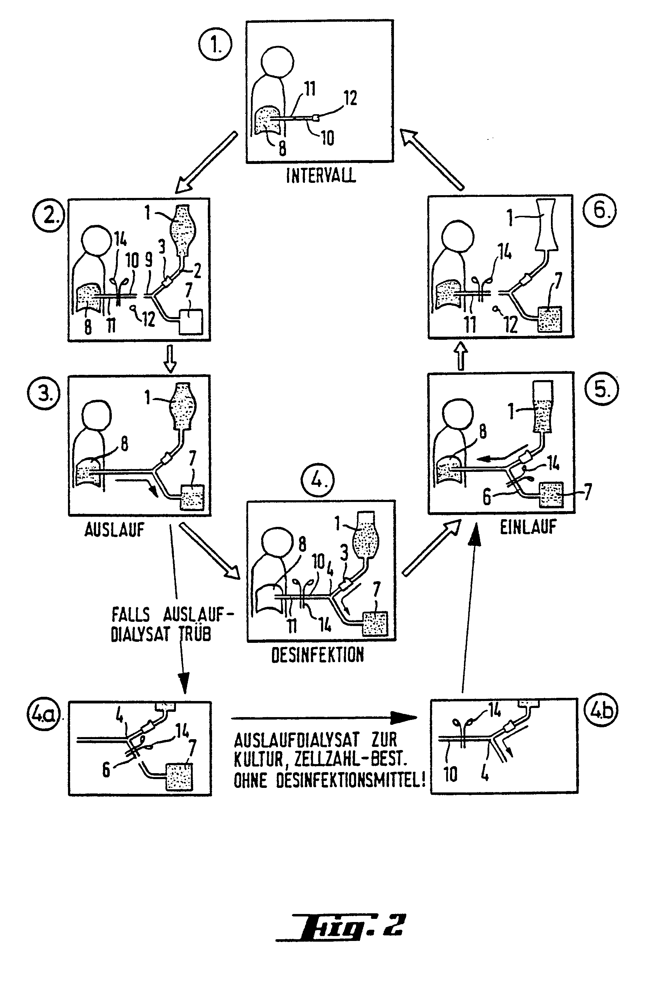

Das Verfahren zur Anwendung des erfindungsgemässen Beutelsystems geht schematisch aus Fig. 2 hervor, wo die verschiedenen Verfahrensschritte in je einer Position dargestellt sind. Pos. 1 zeigt die übliche Situation für den Patienten, dem der Tenckhoff-Katheter 11 in die Bauchhöhle 8 eingesetzt ist. Das Verlängerungsstück 10 ist, sofern überhaupt vorhanden, vorteilhafterweise fest mit dem Katheter 11 verbunden. Entweder der Katheter oder das Verlängerungsstück 10 ist mit einem Sicherheitsverschluss 12 verschlossen. Aus Pos. 2 ersieht man die Vorbereitung für die Dialyse. Der Katheter 11 oder das Verlängerungsstück 10 wird mittels einer Schlauchklemme 14 abgeklemmt, worauf der Sicherheitsverschluss 12 entfernt und an seiner Stelle der dritte Schlauch 9 des Beutelsystems angeschlossen wird. Letzteres weist im ersten Beutel 1 die frische Dialyselösung auf, wogegen der zweite Beutel 7 leer ist. Im ersten Schlauch 2, vor dem Rollschieber 3, befindet sich eine Desinfektionsflüssigkeit.

Nach Anschluss des Beutelsystems wird, wie in Pos. 3 dargestellt, das in der Bauchhöhle 8 des Patienten vorhandene Dialysat in den zweiten Beutel 7 ausfliessen gelassen. Im Normalfall, wenn das Auslaufdialysat klar ist, wird nach vollständiger Entleerung der Bauchhöhle 8 die Verlängerung 10 wieder mittels einer Schlauchklemme 14 verschlossen, bevor der Rollenschieber 3 so lange geöffnet wird (Pos. 4), dass die Desinfektionsflüssigkeit durch das Y-Stück 4 in den zweiten Beutel 7 abfliessen und dabei letzteres desinfizieren kann. Dann wird, wie in Pos. 5 dargestellt, die Schlauchklemme 14 an den zweiten Schlauch 6 gelegt, so dass der zweite Beutel 7 verschlossen und der Weg zur Bauchhöhle 8 wieder frei ist. Jetzt kann das frische Dialysat aus dem ersten Beutel 1 in die Bauchhöhle 8 fliessen, bevor wiederum der Katheter 11 mittels der Schlauchklemme 14 verschlossen wird (Pos. 6), um das Entfernen des Beutelsystems 1, 7 zu ermöglichen. Nachdem das Verlängerungsstück 10 wiederum durch den Sicherheitsverschluss 12 verschlossen worden ist, kann auch die Schlauchklemme 14 wieder entfernt werden, und der Patient kann sich wieder bis zur nächsten Dialyse frei bewegen (Pos. 1).

Sollte festgestellt werden, dass das Auslaufdialysat bei Pos. 3 trüb ist, ist gemäss den Pos. 4a und 4b vorzugehen. Dabei muss, wie aus Pos. 4a ersichtlich, während des Auslaufens des sich in der Bauchhöhle 8 des Patienten befindlichen Dialysats zwischenhinein der zweite Schlauch 6 abgeklemmt werden, so dass eine Probe entnommen werden kann, bevor das Desinfektionsmittel wie in Pos. 4b gezeigt, das Y-Stück 4 reinigt.

Der dank dem erfindungsgemässen Beutelsystem sehr einfach gewordene Dialysatwechsel kann vom Patienten zu Hause durchgeführt werden und erleichtert ihm daher das Leben mit seinem Leiden ganz wesentlich. Durch die Verwendung eines geschlossenen Systems mit Desinfektionslösung kann zudem die Infektionsgefahr wesentlich vermindert werden.

Der Fachmann erkennt leicht, dass das hiervor beschriebene und in der Zeichnung dargestellte Beutelsystem in Einzelheiten anders ausgeführt werden kann. Die Tropfkammer 5 kann gegebenenfalls weggelassen werden, ohne dass dadurch die Funktionsweise des Systems beeinträchtigt würde. Auch sind andere als Luer-Lock-Verbindungsstücke verwendbar. Das Verlängerungsstück 10 ist auch nicht unbedingt erforderlich.

## Patentansprüche

1. Beutelsystem für die Durchführung der ambulanten Peritonealdialyse, umfassend einen ersten Beutel (1), der mit frischem Dialysat gefüllt ist, einen zweiten Beutel (7) für die Aufnahme des gebrauchten Dialysats sowie drei Schläuche (2; 6; 9), von denen der erste Schlauch (2) den ersten Beutel (1), der zweite Schlauch (6) den zweiten Beutel (7) und der dritte Schlauch (9) einen in die Bauchhöhle (8) des Patienten reichenden Katheter (11) je mit einem Anschluss eines Y-Verbindungsstückes (4) verbindet, dadurch gekennzeichnet, dass der erste Schlauch (2) mit Desinfektionslösung gefüllt ist.

2. Beutelsystem nach Anspruch 1, dadurch gekennzeichnet, dass der dritte Schlauch (9) zwischen dem Y-Verbindungsstück (4) und dem Katheter (11) mit einem Luer-Lock versehen ist.

## Claims

1. Bag system for effecting an ambulatory peritoneal dialysis, comprising a first bag (1) filled with fresh dialysate, a second bag (7) for receiving the used dialysate and three tubes (2; 6; 9) from which the first tube (2) connects the first bag (1), the second tube (6) the second bag (7) and the third tube (9) a catheter (11) reaching into the abdominal cavity (8) of the patient each with one branch of a Y-shaped connexion piece (4), characterized in that the first tube (2) is filled with disinfecting solution.

2. Bag system according to claim 1, characterized in that the third tube (9) contains between the Y-shaped connexion piece (4) and the catheter (11) a Luer-Lock.

## Revendications

1. Ensemble de sacs pour l'exécution d'une dialyse péritonéale ambulatoire, comprenant un premier sac (1) rempli avec du dialysat frais, un deuxième sac (7) pour recevoir le dialysat usé ainsi que trois tuyaux (2; 6; 9) dont le premier tuyau (2) raccorde le premier sac (1), le deuxième tuyau (6) le deuxième sac (7) et le troisième tuyau (9) un cathéter (11) pénétrant dans la cavité abdominale (8) du patient, chacun avec une branche d'une pièce de raccord en Y (4), caractérisé en ce que le premier tuyau (2) est rempli avec une solution désinfectante.

2. Ensemble de sacs selon la revendication 1, caractérisé en ce que le troisième tuyau (9) est pourvu d'un Luer-Lock entre la pièce de raccord en Y (4) et le cathéter (11).

**Fig. 1**

**Fig. 2**